# EUROPEAN PATENT SPECIFICATION

(11) **EP 2 919 755 B1**
(45) Date of publication and mention of the grant of the patent: **21.02.2018**
(21) Application number: 13799726.8
(22) Date of filing: 18.11.2013
(51) Int. Cl.: A61K 9/00, A61K 47/14, A61K 47/34, A61K 31/196

(54) **AQUEOUS LIQUID COMPOSITION CONTAINING 2-AMINO-3-(4-BROMOBENZOYL)PHENYLACETIC ACID**
WÄSSRIGE FLÜSSIGZUSAMMENSETZUNG MIT 2-AMINO-3-(4-BROMBENZOYL-)PHENYLESSIGSÄURE
COMPOSITION AQUEUSE LIQUIDE CONTENANT DE L'ACIDE 2-AMINO-3-(4-BROMOBENZOYL)-PHÉNYLACÉTIQUE

(30) Priority: 19.11.2012 US 201261727940 P
(43) Date of publication of application: 23.09.2015
(73) Proprietor: Bausch & Lomb Incorporated, Rochester, NY 14609 (US)
(72) Inventor: PADILLA, Angeliqueo, E., Aliso Viejo, CA 92656 (US); BAKLAYAN, George, A., Huntington Beach, CA 92648 (US)
(74) Representative: Glas, Holger
(86) International application number: PCT/US2013/070510
(87) International publication number: WO 2014/078766

(56) References cited:
- WO-A1-98/52579
- US-B2- 8 129 431

## Description

### TECHNICAL FIELD

The present invention relates to a stable aqueous liquid preparation containing a non-steroidal anti-inflammatory agent. In particular, the present invention relates to a stable aqueous liquid preparation containing 2-amino-3-(4-bromobenzoyl)phenylacetic acid or a pharmacologically acceptable salt thereof or a hydrate thereof. More particularly, the present invention relates to an aqueous liquid preparation containing 2-amino-3-(4-bromobenzoyl)phenylacetic acid or a pharmacologically acceptable salt thereof or a hydrate thereof and a non-ionic surfactant.

### BACKGROUND ART

Benzoylphenylacetic acid derivatives including bromfenac (generic name) of formula (I): 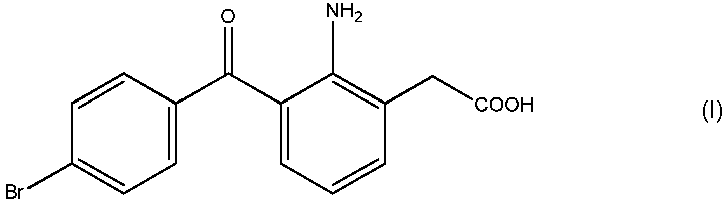 of which chemical name is 2-amino-3-(4-bromobenzoyl)phenylacetic acid are known as disclosed in JP-A-23052/1977 and its corresponding U.S. Pat. No. 4,045,576. 2-Amino-3-(4-bromobenzoyl)phenylacetic acid, its pharmacologically acceptable salt and a hydrate thereof are known as a non-steroidal anti-inflammatory agent, and they are effective against inflammatory diseases of anterior or posterior segment of the eye, such as blepharitis, conjunctivitis, scleritis, and postoperative inflammation in the field of ophthalmology, and its sodium salt has been practically used in the form of eye drops ("New Drugs in Japan, 2001", 2001 Edition, Published by Yakuji Nippo Ltd., May 11, 2001, p. 27-29).

It was known that 2-amino-3-(4-bromobenzoyl)phenylacetic acid was unstable in aqueous formulations. Therefore, efforts were made to stabilize the eye drop mentioned above by means of addition of a watersoluble polymer (e.g., polyvinylpyrrolidone, polyvinyl alcohol, etc.) and a sulfite (e.g., sodium sulfite, potassium sulfite, etc.) (Japanese Patent No. 2,683,676 and its corresponding U.S. Pat. No. 4,910,225). Data were presented for only polyvinylpyrrolidone and polysorbate 80. It appeared that the formulations were stable when the pH was 8 or higher. However, even then, red precipitates were observed with formulations having polysorbate 80 after 3 weeks.

In addition, as an eye drop other than the above-mentioned one, Japanese Patent No. 2,954,356 (corresponding to U.S. Pat. Nos. 5,603,929 and 5,653,972) discloses an ophthalmic composition which comprises incorporating the antibacterial quaternary ammonium polymer Polyquad® (also known generically as polyquat-1) and boric acid into a formulation containing diclofenac as an acidic ophthalmic agent. Data was presented to show that the preservative efficacy of Polyquad® was maintained in one formulation. Although bromfenac was mentioned among the ophthalmic agents, no data was presented for any formulation containing that compound. The patentee there spoke of the stability of the formulations in term of the maintenance of the preservative efficacy. Nothing was disclosed regarding the physical or chemical stability of the active compound.

In Japanese Patent No. 2,954,356, there is the following description -- "Benzalkonium chloride is a widely used preservative in ophthalmic solutions. However, benzalkonium chloride and other quaternary ammonium compounds are generally considered to be incompatible with ophthalmic compositions of drugs with acidic groups, such as nonsteroidal anti-inflammatory drugs. These preservatives lose their ability to function as they form complexes with the charged drug compounds."

In these references, there is no disclosure or suggestion of, or the manner for, maintaining preservative efficacy and stability of the pharmaceutical active ingredient.

Furthermore, U.S. patent 8 129 431 B2 refers to a stable aqueous liquid containing 2-amino-3-(4-bromobenzoyl)phenylacetic acid or its pharmacologically acceptable salt or a hydrate thereof, an alkyl aryl polyether alcohol type polymer such as tyloxapol, or a polyethylene glycol fatty acid ester such as polyethylene glycol monostearate. International patent application WO 98/52579 A1 refers to an ophthalmic composition containing a divalent salt and a non-steroidal anti-inflammatory agent as a precipitate.

### SUMMARY

In one aspect, the present invention provides an aqueous liquid composition comprising: (a) 2-amino-3-(4-bromobenzoyl)phenylacetic acid or a pharmacologically acceptable salt thereof or a hydrate thereof; (b) a non-ionic surfactant; (c) water; and (d) an ophthalmically acceptable preservative selected from the group consisting of polyaminopropyl biguanide ("PAPB"), polyquaternium-1, perborate, and mixtures thereof; wherein the non-ionic surfactant is selected from the group consisting of Poloxamer 407, Octoxynol 40, PEG40 hydrogenated castor oil, and mixtures thereof; wherein at least 90 percent of the original amount of said 2-amino-3-(4-bromobenzoyl)phenylacetic acid or pharmacologically acceptable salt thereof or hydrate thereof remains in the composition after storage at 60 °C, for 4 weeks; and wherein the composition satisfies preservative efficacy acceptance criteria of US Pharmacopeia 35 (2012).

In another aspect, the invention provides a method for inhibiting decrease in preservative efficacy of an ophthalmically acceptable preservative in an aqueous composition that comprises: (a) 2-amino-3-(4-bromobenzoyl)phenylacetic acid or a pharmacologically acceptable salt thereof or a hydrate thereof; and (b) an ophthalmically acceptable preservative selected from the group consisting of polyaminopropyl biguanide ("PAPB"), polyquaternium-1, perborate, and mixtures thereof; which method comprises incorporating a non-ionic surfactant into the aqueous liquid composition; wherein the non-ionic surfactant is selected from the group consisting of Poloxamer 407, Octoxynol 40, PEG40 hydrogenated castor oil, and mixtures thereof, and wherein the preservative efficacy satisfies the preservative efficacy acceptance criteria of US Pharmacopeia 35 (2012).

### DETAILED DESCRIPTION

As a result of various studies, the inventors of the present invention have found that, by adding certain non-ionic surfactant to an aqueous liquid composition containing a pharmaceutical active ingredient ("API") of 2-amino-3-(4-bromobenzoyl)phenylacetic acid or a pharmacologically acceptable salt thereof or a hydrate thereof, degradation of said API in said aqueous composition is inhibited.

According to the present invention, at least 90 percent (alternatively, at least 92 or at least 94 percent) of the original amount of said API remains in the composition after storage at 60 °C, for 4 weeks. In one embodiment, such storage is carried out in ambient humidity.

For example, an aqueous liquid composition may comprise 2-amino-3-(4-bromobenzoyl)phenylacetic acid or a pharmacologically acceptable salt thereof or a hydrate thereof, a non-ionic surfactant, and water. The non-ionic surfactant may be selected from the group consisting of copolymers of polyoxyethylene and polyoxypropylene, fatty acid esters of polyethylene glycol, polyethylene glycol mono(alkylaryl) ethers, polyethoxylated triglycerides, polyethoxylated hydrogenated triglycerides, and mixtures thereof, and at least 90 percent (alternatively, at least 92 or at least 94 percent) of the original amount of said 2-amino-3-(4-bromobenzoyl)phenylacetic acid or pharmacologically acceptable salt thereof or hydrate thereof remains in the composition after storage at 60 °C, for 4 weeks.

Such an aqueous liquid preparation may not include polysorbate 80.

Another exemplary aqueous liquid composition comprise 2-amino-3-(4-bromobenzoyl)phenylacetic acid or a pharmacologically acceptable salt thereof or a hydrate thereof, a non-ionic surfactant, and water, wherein the non-ionic surfactant is selected from the group consisting of copolymers of polyoxyethylene and polyoxypropylene, fatty acid esters of polyethylene glycol, polyethylene glycol mono(alkylaryl) ethers, polyethoxylated triglycerides, polyethoxylated hydrogenated triglycerides, and mixtures thereof.

Yet another exemplary aqueous liquid composition comprise 2-amino-3-(4-bromobenzoyl)phenylacetic acid or a pharmacologically acceptable salt thereof or a hydrate thereof, a non-ionic surfactant, water, and an ophthalmically acceptable preservative. The non-ionic surfactant may be a polyethylene glycol poly(alkylaryl) ether, and at least 90 percent (alternatively, at least 92 or at least 94 percent) of the original amount of said 2-amino-3-(4-bromobenzoyl)phenylacetic acid or pharmacologically acceptable salt thereof or hydrate thereof remains in the composition after storage at 60 °C, for 4 weeks.

A further example of such an aqueous liquid composition may comprise 2-amino-3-(4-bromobenzoyl)phenylacetic acid or a pharmacologically acceptable salt thereof or a hydrate thereof, a non-ionic surfactant, water, and benzalkonium chloride; wherein the non-ionic surfactant is selected from the group consisting of copolymers of polyoxyethylene and polyoxypropylene, polyethylene glycol mono(alkylaryl) ethers, polyethoxylated triglycerides, and mixtures thereof; and wherein at least 90 percent (alternatively, at least 92 or at least 94 percent) of the original amount of said 2-amino-3-(4-bromobenzoyl)phenylacetic acid or pharmacologically acceptable salt thereof or hydrate thereof remains in the composition after storage at 60 °C, for 4 weeks.

Still another example may be an aqueous liquid composition of the present invention comprising 2-amino-3-(4-bromobenzoyl)phenylacetic acid or a pharmacologically acceptable salt thereof or a hydrate thereof, a non-ionic surfactant, water, and an ophthalmically acceptable preservative; wherein the non-ionic surfactant is a polyethylene glycol poly(alkylaryl) ether; and wherein the ophthalmically acceptable preservative is selected from the group consisting of chlorhexidine, polyaminopropyl biguanide ("PAPB"), polyquaternium-1, polyquaternium-42, perborate, and mixtures thereof. The polyethylene glycol poly(alkylaryl) ether may be tyloxapol.

Non-limiting examples of non-ionic surfactants of the type of copolymers of polyoxyethylene and polyoxypropylene are known by their chemical names of α-hydro-ω-hydroxypoly(oxyethylene)*ₐ*poly(oxypropylene)*_{b}*poly(oxyethylene)*ₐ* block copolymer having formula of HO(CH₂CH₂O)*ₐ*(CH₂CH₂CH₂O)*_{b}*(CH₂CH₂O)*ₐ*H; wherein
a is in the range of 10-120, and b is in the range of 20-60. Various surfactants in this family are known under their common names of poloxamer, such as poloxamer 124, 188, 236, 338, and 407.

| Poloxamer | *a* | *b* |
|---|---|---|
| 124 | 12 | 20 |
| 188 | 80 | 27 |
| 237 | 64 | 37 |
| 338 | 141 | 44 |
| 407 | 101 | 56 |

Non-limiting examples of fatty acid esters of polyethylene glycol include esters of fatty acids having 8-24 carbon atoms (caprylic acid, capric acid, lauric acid, myristic acid, palmitic acid, stearic acid, arachic acid, behenic acid, lignoceric acid, oleic acid, linoleic acid, linolenic acid) and polyethylene glycol having 6-60 repeating units of -CH₂CH₂O-. A mono-fatty acid ester of polyethylene glycol is, for example, polyoxyl 40 stearate.

Non-limiting examples of polyethylene glycol mono(alkylaryl) ethers include polyethylene nonylphenyl ethers (having common name of Nonoxynol or Nonoxinol; e.g., Nonoxinol 9, 11, and 11) and polyethylene glycol tetramethylbutylphenyl ethers (having common name of Octoxynol or Octoxinol; e.g., Octoxynol 9, 10, 16, 20, 30, and 40). A polyethylene glycol mono(alkylaryl) ether is, for example, Octoxynol 40.

A non-limiting example of polyethylene glycol poly(alkylaryl) ether is tyloxapol, having Formula II.

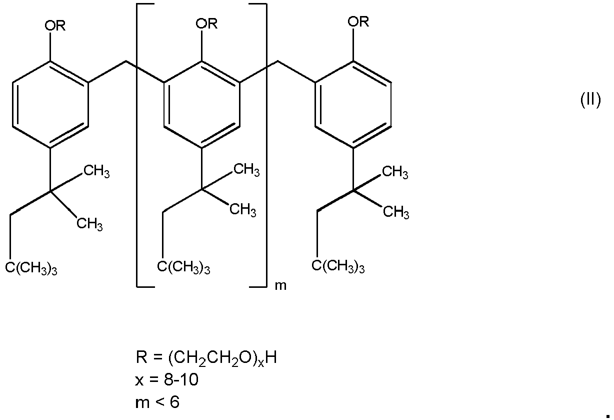

Non-limiting examples of polyethoxylated triglycerides and polyethoxylated hydrogenated triglycerides include polyoxyl N castor oils (wherein N = 30-40, such as polyoxyl 35 castor oil and polyoxyl 40 castor oil) and polyoxyl N hydrogenated castor oils (wherein N = 4-60, such as polyoxyl 40 castor oil and polyoxyl 60 castor oil). A non-ionic surfactant is, for example, polyoxy 40 castor oil.

As a result of various studies, the inventors of the present invention have found that, by adding a certain non-ionic surfactant such as one disclosed herein to an aqueous liquid preparation of 2-amino-3-(4-bromobenzoyl)phenylacetic acid or a pharmacologically acceptable salt thereof or a hydrate thereof, the aqueous solution becomes stable within a pH range, and change in the amount of the 2-amino-3-(4-bromobenzoyl)phenylacetic acid over time can be inhibited. Further, when the aqueous solution contains a preservative, deterioration in the preservative effect of said preservative can be inhibited for a significant period of time, such as at least 28 days. In addition, the inventive preparation can reduce or avoid irritation to the eyes.

Namely, the present invention provides:
(1) An aqueous liquid composition comprising: (a) 2-amino-3-(4-bromobenzoyl)phenylacetic acid or a pharmacologically acceptable salt thereof or a hydrate thereof; (b) a non-ionic surfactant; (c) water; and (d) an ophthalmically acceptable preservative selected from the group consisting of polyaminopropyl biguanide ("PAPB"), polyquaternium-1, perborate, and mixtures thereof; wherein the non-ionic surfactant is selected from the group consisting of Poloxamer 407, Octoxynol 40, PEG40 hydrogenated castor oil, and mixtures thereof; wherein at least 90 percent of the original amount of said 2-amino-3-(4-bromobenzoyl)phenylacetic acid or pharmacologically acceptable salt thereof or hydrate thereof remains in the composition after storage at 60 °C, for 4 weeks; and wherein the composition satisfies preservative efficacy acceptance criteria of US Pharmacopeia 35 (2012).
(2) The aqueous liquid preparation according to (1), wherein the concentration of the non-ionic surfactant is in the range from 0.005 w/v % to 0.12 w/v%.
(3) The aqueous liquid preparation according to any one of the above (1) to (2), wherein the concentration of the non-ionic surfactant is in the range from 0.007 w/v % to 0.1 w/v %.
(4) The aqueous liquid preparation according to any one of the above (1) to (3), wherein the concentration of the non-ionic surfactant is in the range from 0.01 w/v % to 0.07 w/v %.
(5) The aqueous liquid preparation according to any one of the above (1) to (4), wherein the concentration of the 2-amino-3-(4-bromobenzoyl)phenylacetic acid or a pharmacologically acceptable salt thereof or a hydrate thereof is 0.01 to 0.5 w/v %.
(6) The aqueous liquid preparation according to any one of the above (1) to (5), wherein the concentration of the 2-amino-3-(4-bromobenzoyl)phenylacetic acid or a pharmacologically acceptable salt thereof or a hydrate thereof is 0.01 to 0.2 w/v %.
(7) The aqueous liquid preparation according to any one of the above (1) to (6), wherein the concentration of the 2-amino-3-(4-bromobenzoyl)phenylacetic acid or a pharmacologically acceptable salt thereof or a hydrate thereof is 0.01 to 0.1 w/v %.
(8) The aqueous liquid preparation according to any one of the above (1) to (7), wherein the concentration of the 2-amino-3-(4-bromobenzoyl)phenylacetic acid or a pharmacologically acceptable salt thereof or a hydrate thereof is 0.05 to 0.1 w/v %.
(9) The aqueous liquid preparation according to any one of the above (1) to (8), wherein the pharmacologically acceptable salt of 2-amino-3-(4-bromobenzoyl)phenylacetic acid is a sodium salt.
(10) The aqueous liquid preparation according to any one of the above (1) to (9), wherein the pH of the aqueous liquid preparation is within a range of 7 to 9.
(11) The aqueous liquid preparation according to the above (1) to (10), wherein the pH of the aqueous liquid preparation is within a range of 7.3 to 8.5.
(12) The aqueous liquid preparation according to the above (1) to (11), wherein the pH of the aqueous liquid preparation is within a range of 7.4 to 8.3.
(13) The aqueous liquid preparation according to any one of the above (1) to (12), wherein the aqueous liquid preparation is an eye drop.
(14) The aqueous liquid preparation according to any one of the above (1) to (13), wherein the aqueous liquid preparation is a nasal drop.
(15) A method for inhibiting decrease in preservative efficacy of an ophthalmically acceptable preservative in an aqueous composition that comprises: (a) 2-amino-3-(4-bromobenzoyl)phenylacetic acid or a pharmacologically acceptable salt thereof or a hydrate thereof; and (b) an ophthalmically acceptable preservative selected from the group consisting of polyaminopropyl biguanide ("PAPB"), polyquaternium-1, perborate, and mixtures thereof; which method comprises incorporating a non-ionic surfactant into the aqueous liquid composition; wherein the non-ionic surfactant is selected from the group consisting of Poloxamer 407, Octoxynol 40, PEG40 hydrogenated castor oil, and mixtures thereof, and wherein the preservative efficacy satisfies the preservative efficacy acceptance criteria of US Pharmacopeia 35 (2012).
(16) A liquid preparation, as recited in any one of the above (1) to (14), wherein the liquid preparation, composition, or formulation does not include any polyol.
(17) The liquid preparation of the above (16), wherein the polyol is mannitol.

According to the present invention, a stable aqueous liquid preparation containing 2-amino-3-(4-bromobenzoyl)phenylacetic acid or a pharmacologically acceptable salt thereof or a hydrate thereof can be prepared by incorporating a non-ionic surfactant, chosen from those disclosed in claim 1, into an aqueous liquid preparation containing 2-amino-3-(4-bromobenzoyl)phenylacetic acid or a pharmacologically acceptable salt thereof or a hydrate thereof. Also, an aqueous liquid preparation of the present invention, wherein a preservative as defined in claim 1 is incorporated and has a sufficient preservative efficacy. According to the present invention, such preservative efficacy satisfies the acceptance criteria of US Pharmacopeia 35 (2012).

Therefore, the aqueous liquid preparation of the present invention is advantageously used as an eye drop for the treatment of, for example, blepharitis, conjunctivitis, scleritis, and postoperative inflammation. In addition, such aqueous liquid preparation can be used as a nasal drop for the treatment of, for example, allergic rhinitis and inflammatory rhinitis (e.g., chronic rhinitis, hypertrophic rhinitis, nasal polyp, etc.).

The pharmacologically acceptable salt of 2-amino-3-(4-bromobenzoyl)phenylacetic acid includes, for example, an alkali metal salt such as sodium salt and potassium salt, and an alkaline earth metal salt such as calcium salt and magnesium salt, among which sodium salt is especially preferable.

2-Amino-3-(4-bromobenzoyl)phenylacetic acid and its pharmacologically acceptable salt can be prepared according to the method as described in JP-A-23052/1977 (corresponding to U.S. Pat. No. 4,045,576) or by a similar method thereof. These compounds can be obtained as their hydrate depending on synthetic conditions and recrystallization conditions. The hydrate includes 1/2 hydrate, 1 hydrate, and 3/2 hydrate, among which 3/2 hydrate is preferable.

In the aqueous liquid preparation of the present invention, the content (concentration range) of 2-amino-3-(4-bromobenzoyl)phenylacetic acid or a pharmacologically acceptable salt thereof or a hydrate thereof is usually about 0.01 to 0.5 w/v %, or about 0.03 to 0.2 w/v %, or about 0.05 to 0.2 w/v %, or about 0.05 to 0.1 w/v %, or about 0.06 to about 0.1 w/v %. The concentration can appropriately vary to have any numerical value in said ranges, depending on the purpose of use and the degree of disease to be treated.

As used herein, the "alkyl" group can have a carbon number in the range from 1 to 18. For example, the alkyl group includes methyl, ethyl, propyl, isopropyl, cyclopropyl, butyl, isobutyl, sec-butyl, tert-butyl, cyclobutyl, pentyl, isopentyl, neopentyl, tert-pentyl, 1-ethylpropyl, 4-methylpentyl, 1,1-dimethylbutyl, 2,2-dimethylbutyl, 1,2-dimethylbutyl, 2-ethylbutyl, cyclopentyl, hexyl, cyclohexyl, heptyl, isoheptyl, octyl, isooctyl, nonyl, isononyl, decyl, isodecyl, undecyl, isoundecyl, dodecyl, isododecyl, tridecyl, isotridecyl, tetradecyl, isotetradecyl, pentadecyl, isopentadecyl, hexadecyl, isohexadecyl, heptadecyl, isoheptadecyl, octadecyl, isooctadecyl, and isomers thereof. For example, the octyl group includes isooctyl, sec-octyl, 1-methylheptyl, 1-ethylhexyl, 2-ethylhexyl, 1-propylpentyl, 1,5-dimethylhexyl, and 1,1,3,3-tetramethylbutyl.

As used herein, the "aryl" group includes one or more fused or bridged rings having a total carbon number from 5 to 14. Preferably, the aryl group is the phenyl group.

The polyoxyethylene group can be represented by the formula O(CH₂CH₂O)ₓH in which x is an integer from 5 to 100, inclusive; such as 5 to 60, or 5 to 40, or 5 to 30.

The fatty acid of a polyethylene glycol fatty acid ester can be a fatty acid having the carbon number of 8 to 24. Non-limiting examples of such polyethylene glycol fatty acid esters are polyethylene glycol monostearate (e.g. polyoxyl 8 stearate, polyoxyl 40 stearate, etc.), polyethylene glycol monolaurate, polyethylene glycol monooleate, polyethylene glycol diisostearate, polyethylene glycol dilaurate, polyethylene glycol dioleate, and the like. In some embodiments, polyoxyl 40 stearate is used. Polyoxyl 40 stearate is an non-ionic surfactant and is a monostearic acid ester of an ethylene oxide condensed polymer. It can be represented by the formula C₁₇H₃₅COO(CH₂CH₂O)ₙH, wherein n is about 40.

Although the concentration range of the non-ionic surfactant in the aqueous liquid preparation of the present invention depends on the kind of compounds used, the minimum concentration is about 0.01 w/v % and the maximum concentration is about 0.5 w/v %. For example, the concentration range of a non-ionic surfactant can be from 0.01 w/v % to 0.05 w/v %, from 0.01 w/v % to 0.07 w/v, from 0.01 w/v % to 0.1 w/v %, from 0.01 w/v % to 0.12 w/v %, from 0.02 w/v % to 0.05 w/v %, from 0.02 w/v % to 0.07 w/v, from 0.02 w/v % to 0.1 w/v %, from 0.02 w/v % to 0.12 w/v %, from 0.03 w/v % to 0.05 w/v %, from 0.03 w/v % to 0.07 w/v, from 0.03 w/v % to 0.1 w/v %, or from 0.03 w/v % to 0.12 w/v %.

The incorporation ratio of a non-ionic surfactant in the aqueous liquid preparation of the invention is within a range from about 0.1 to about 0.5, from about 0.1 to about 1, or from about 0.5 to about 3, or from about 0.5 to about 5, or from about 0.2 to about 0.5, from about 0.2 to about 1, or from about 0.2 to about 3, or from about 0.3 to about 5, parts by weight relative to 1 part by weight of 2-amino-3-(4-bromobenzoyl)phenylacetic acid or its pharmacologically acceptable salt or a hydrate thereof.

In some embodiments, when the preservative is polyquaternium-1 (also known as Polyquad®), a preparation, composition, or formulation of the present invention excludes polyols. In one aspect, such preparation, composition, or formulation can still satisfy the preservative efficacy acceptance criteria of US Pharmacopeia 35 (2012).

Further, so long as the purpose of the present invention is achieved, various conventional additives such as tonicity-adjusting agents, buffers, thickeners, stabilizers, chelating agents, pH controlling agents, perfumes and the like may be appropriately added to the aqueous liquid preparation of the present invention. Non-limiting examples of tonicity-adjusting agents include sodium chloride, potassium chloride, monosaccharides (such as glucose), or disaccharides (such as sucrose). The buffers include, for example, phosphate buffer, borate buffer, citrate buffer, tartrate buffer, acetate buffer, boric acid, borax, amino acids, and the like. The thickeners include polyvinylpyrrolidone, carboxymethylcellulose, carboxypropylcellulose, hydroxyethylcellulose, hydroxypropylcellulose, hydroxypropylmethylcellulose, polyvinyl alcohol, sodium polyacrylate, and the like. The stabilizers include sulfites such as sodium sulfite and the like. The chelating agents include sodium edetate, sodium citrate, sodium phosphate and the like. The pH controlling agents include hydrochloric acid, sodium hydroxide, phosphoric acid, acetic acid and the like. The perfumes include 1-menthol, borneol, camphor, Eucalyptus oil, and the like.

With respect to the concentrations of the above various additives in the aqueous liquid preparation of the present invention, a tonicity-adjusting agent may be included to achieve an osmolality in the range from about 230 to 350 mOsmol/kg (or from about 270 to about 320 mOsmol/kg).

The concentration of the buffering agent is chosen appropriately to achieve the desired pH, depending on the buffering agent. Such choice is within the skill of the art. For example, the buffering agent concentration can be in the range of 0.01 to 2 w/v %.

The concentration of a thickener to be added can be in the range from 0.1 to 10 w/v %, or from 0.1 to 3 w/v%, or from 0.1 to 1 w/v%.

The pH of the aqueous liquid preparation of the present invention is adjusted to about 6 to 9, or about 7 to 9, or about 7.3 to 8.5, or about 7.4 to 8.3.

So long as the purpose of the present invention is achieved, other kinds of active pharmaceutical ingredients may be appropriately added to treat or ameliorate additional conditions or disorders if such other active pharmaceutical ingredients are compatible with bromfenac or its pharmaceutically acceptable salts or esters (physically, chemically, and physiologically).

The aqueous liquid preparation of the present invention can be prepared by per se known method or according to the method as described in the Japanese Pharmacopoeia, 14th Edition, General Rules for Preparations, Solutions or Ophthalmic solutions.

For example, all desired ingredients at appropriate amounts (to achieve the final concentrations) may be added to a sterilized vessel, equipped with appropriate temperature control, internal atmosphere control, purging, and mixing implements. The ingredients may be added sequentially or together. The contents of the vessel are then mixed thoroughly until the contents are uniform. The contents are then packaged under sterility conditions into individual units.

The aqueous liquid preparation of the present invention can be applied to warm-blooded animals such as human, rat, mouse, rabbit, cow, pig, dog, cat, and the like.

The aqueous liquid preparation of the present invention can be prepared easily by dissolving the above-mentioned components in, for example, distilled water or sterile purified water. For example, the aqueous liquid preparation in the form of an eye drop can be used for the treatment of inflammatory diseases in anterior or posterior segment of the eye such as blepharitis, conjunctivitis, scleritis, postoperative inflammation, and the like. The dose of the aqueous liquid preparation containing 0.1 w/v % of sodium 2-amino-3-(4-bromobenzoyl)phenylacetate hydrate is, for example, administered to an adult 3 to 6 times daily in an amount of 1 to 2 drops per one time. Depending on the degree of diseases, frequency of dosing is appropriately controlled.

The present invention is illustrated by way of non-limiting examples disclosed below.

Tables 1, 2, 3, 4, 5, and 6 present non-limiting exemplary compositions of the present invention. Tables 1 R, 2R, 3R, 4R, 5R, and 6R present the results of stability and preservative efficacy testing of these compositions.

Tables 1R, 2R, 3R, 4R, and 5R show the remaining amount of sodium 2-amino-3-(4-bromobenzoyl)phenylacetate after storage at 60° C. for 4 weeks as the percentage of the original amount of sodium 2-amino-3-(4-bromobenzoyl)phenylacetate at the time of production. Correction for moisture vaporization from the container was also taken into account. As is apparent from these Tables, the remaining amount of sodium 2-amino-3-(4-bromobenzoyl)phenylacetate in these compositions indicate that bromfenac is surprisingly stable in a wide variety of compositions comprising various non-ionic surfactants and preservatives. These results indicate that these exemplary compositions or some variations thereof have good stability to be considered for use as pharmaceutical products, such as eye drops.

Preservative efficacy testing of these compositions was carried out against *Staphylococcus aureus* (hereinafter referred to as *S*. *aureus*), *Escherichia Coli* (hereinafter referred to as *E. coli*), *Pseudomonas aeruginosa* (hereinafter referred to as *P. aeruginosa*), *Candida albicans* (hereinafter referred to as *C. albicans*) and *Aspergillus brasiliensis* (hereinafter referred to as *A. brasiliensis*).

The results are shown in Tables 1R, 2R, 3R, 4R, 5R, and 6R.

The EP-criteria A and EP-criteria B for preservative efficacy are given in the following.

### EP-Criteria A (2012):

Viable cell counts of bacteria (*S. aureus, P. aeruginosa*) 6 hours, 24 hours, and 28 days after inoculation decrease to not more than 1/100, not more than 1/1000, and undetectable, respectively.

Viable cell count of fungi (*C*. *albicans, A. brasiliensis*) 7 days after inoculation decreases to not more than 1/100, and thereafter, the cell count levels off or decreases.

### EP-Criteria B (2012):

Viable cell counts of bacteria (*S*. *aureus, P. aeruginosa*) at 24 hours and 7 days after inoculation decrease to not more than 1/10 and not more than 1/1000, respectively, and thereafter, the cell counts level off or decrease.

Viable cell counts of fungi (*C*. *albicans, A. brasiliensis*) at 14 days after inoculation decrease to not more than 1/10, and thereafter, the cell counts keep the same level as that of 14 days after inoculation.

The USP (2012) criteria for ophthalmic products are given in the following.

Viable cell counts of bacteria (*E. coli, S. aureus, P. aeruginosa*) decrease to not more than 1/10 (of the initial counts) at 7 days after initial inoculation, not more than 1/1000 (of the initial counts) at 14 days after the initial inoculation, and at 28 days do not increase from the 14 days' cell counts.

Viable cell counts of fungi (*C*. *albicans, A. brasiliensis*) do not increase from the initial calculated cell counts at 7, 14, and 28 days after the initial inoculation.

**Table 1. Non-Limiting Preparations of the Present Invention Including Representative Preservatives**

| Ingredient | NGB-1 (%w/v) | NGB-2 (%w/v) | NGB-3 (%w/v) | NGB-4 (%w/v) | NGB-5 (%w/v) | NGB-1 A (%w/v) |
|---|---|---|---|---|---|---|
| Bromfenac | 0.07 | 0.07 | 0.07 | 0.07 | 0.07 | 0.07 |
| Boric Acid | 1.4 | 1.4 | 1.4 | 1.4 | 1.4 | 1.4 |
| Sodium Borate | 0.74 | 0.74 | 0.74 | 0.74 | 0.74 | 0.74 |
| Sodium Sulfite | 0.2 | 0.2 | 0.2 | 0.2 | 0.2 | 0.2 |
| EDTA | 0.02 | 0.02 | 0.02 | 0.02 | 0.02 | 0.02 |
| Povidone | 1.00 | 1.00 | 1.00 | 1.00 | 1.00 | 1.00 |
| Tyloxapol | 0.02 | 0.02 | 0.02 | 0.02 | 0.02 | 0.02 |
| 2N NaOH | pH to 7.8 | pH to 7.8 | pH to 7.8 | pH to 7.8 | pH to 7.8 | pH to 8.3 |
| Polyquaternium-1 | 0.001 | --- | --- | --- | --- | 0.001 |
| Polyquaternium-42 | --- | 0.003 | --- | --- | --- | --- |
| Sodium Perborate | --- | --- | 0.02 | | --- | --- |
| Dequest 2060^{a} | | | 0.005 | | | |
| PAPB | --- | --- | --- | 0.0001 | --- | --- |
| chlorhexidine | --- | --- | --- | --- | 0.005 | --- |

| | | | | | | |
|---|---|---|---|---|---|---|
| ^{a}Perborate generates a low concentration of hydrogen peroxide that requires phosphonates for stability | | | | | | |

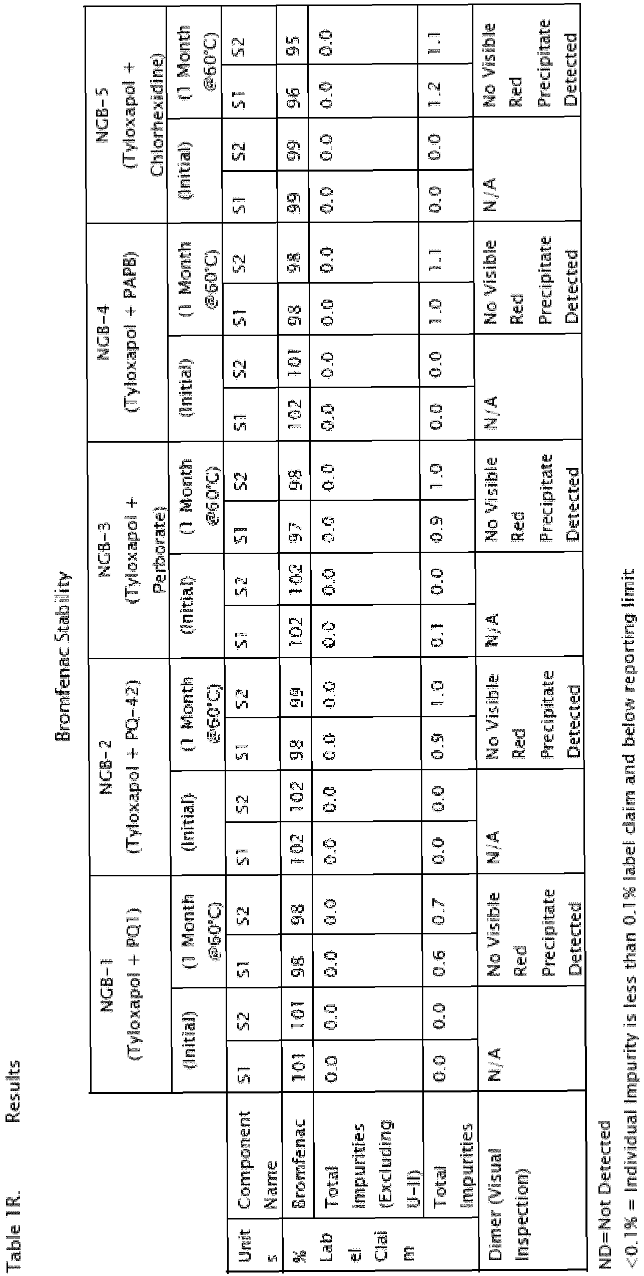

### Preservative Efficacy

**NGB-1**

| Organism | Time Intervals | | | | | | |
|---|---|---|---|---|---|---|---|
| | 0 hr | 6 hr | 24 hr | 48 hr | 7 day | 14 day | 28 day |
| *A. brasiliensis* | N/A | N/A | N/A/ | N/A | ∼3.32 | ∼3.54 | >4.10 |
| *C. Albicans* | N/A | N/A | N/A/ | N/A | >4.53 | >4.53 | >4.53 |
| *E. coli* | >4.45 | N/A | >4.45 | N/A | >4.45 | >4.45 | >4.45 |
| *S. aureus* | >4.13 | N/A | >4.13 | N/A | >4.13 | >4.13 | >4.13 |
| *P. aeruginosa* | >4.02 | N/A | >4.02 | N/A | >4.02 | >4.02 | >4.02 |

| | | | | | | | |
|---|---|---|---|---|---|---|---|
| N/A = not available because there is no requirement at this time point | | | | | | | |

**NGB-2**

| Organism | Time Intervals | | | | | | |
|---|---|---|---|---|---|---|---|
| | 0 hr | 6 hr | 24 hr | 48 hr | 7 day | 14 day | 28 day |
| *A. brasiliensis* | N/A | N/A | N/A/ | N/A | ∼3.16 | ∼3.28 | >4.10 |
| *C. Albicans* | N/A | N/A | N/A/ | N/A | >4.53 | >4.53 | >4.53 |
| *E. coli* | >4.45 | N/A | >4.45 | N/A | >4.45 | >4.45 | >4.45 |
| *S. aureus* | >4.13 | N/A | >4.13 | N/A | >4.13 | >4.13 | >4.13 |
| *P. aeruginosa* | >4.02 | N/A | >4.02 | N/A | >4.02 | >4.02 | >4.02 |

**NGB-3**

| Organism | Time Intervals | | | | | | |
|---|---|---|---|---|---|---|---|
| | 0 hr | 6 hr | 24 hr | 48 hr | 7 day | 14 day | 28 day |
| *A. brasiliensis* | N/A | N/A | N/A/ | N/A | 2.65 | 3.18 | 2.92 |
| *C. Albicans* | N/A | N/A | N/A/ | N/A | ∼3.69 | >4.53 | >4.53 |
| *E. coli* | 0.79 | N/A | 2.46 | N/A | >4.45 | >4.45 | >4.45 |
| *S. aureus* | -0.12 | N/A | -0.03 | N/A | >4.13 | >4.13 | >4.13 |
| *P. aeruginosa* | 0.12 | N/A | 2.06 | N/A | >4.02 | >4.02 | >4.02 |

**NGB-4**

| Organism | Time Intervals | | | | | | |
|---|---|---|---|---|---|---|---|
| | 0 hr | 6 hr | 24 hr | 48 hr | 7 day | 14 day | 28 day |
| *A. brasiliensis* | N/A | N/A | N/A/ | N/A | 2.68 | 2.73 | ∼3.28 |
| *C. Albicans* | N/A | N/A | N/A/ | N/A | ∼4.53 | >4.53 | >4.53 |
| *E. coli* | ∼3.01 | N/A | >4.45 | N/A | >4.45 | >4.45 | >4.45 |
| *S. aureus* | 0.61 | N/A | >4.13 | N/A | >4.13 | >4.13 | >4.13 |
| *P. aeruginosa* | 2.39 | N/A | >4.02 | N/A | >4.02 | >4.02 | >4.02 |

**NGB-5**

| Organism | Time Intervals | | | | | | |
|---|---|---|---|---|---|---|---|
| | 0 hr | 6 hr | 24 hr | 48 hr | 7 day | 14 day | 28 day |
| *A. brasiliensis* | N/A | N/A | N/A/ | N/A | >4.10 | ∼4.10 | >4.10 |
| *C. Albicans* | N/A | N/A | N/A/ | N/A | ∼4.53 | >4.53 | >4.53 |
| *E. coli* | >4.45 | N/A | >4.45 | N/A | >4.45 | >4.45 | >4.45 |
| *S. aureus* | 0.54 | N/A | >4.13 | N/A | >4.13 | >4.13 | >4.13 |
| *P. aeruginosa* | ∼4.02 | N/A | >4.02 | N/A | >4.02 | >4.02 | >4.02 |

**NGB-1 A**

| Organism | Time Intervals | | | | | | |
|---|---|---|---|---|---|---|---|
| | 0 hr | 6 hr | 24 hr | 48 hr | 7 day | 14 day | 28 day |
| *A. brasiliensis* | N/A | N/A | N/A/ | N/A | 2.73 | ∼3.32 | ∼3.88 |
| *C. Albicans* | N/A | N/A | N/A/ | N/A | ∼4.53 | >4.53 | >4.53 |
| *E. coli* | ∼3.01 | N/A | >4.45 | N/A | >4.45 | >4.45 | >4.45 |
| *S. aureus* | 0.61 | N/A | >4.13 | N/A | >4.13 | >4.13 | >4.13 |
| *P. aeruginosa* | 2.39 | N/A | >4.02 | N/A | >4.02 | >4.02 | >4.02 |

Bromfenac Is Chemically Stable in Compositions of the Present Invention in the Presence of a Wide Variety of Preservatives.

Table 1 shows compositions comprising the non-ionic surfactant tyloxapol and exemplary preservatives other than benzalkonium chloride ("BAK"): polyquat-1, polyquat-42, perborate/Dequest® 2060, polyaminopropyl biguanide, and chlorhexidine. The chemical stability is judged from the remaining amount of bromfenac in the formulation after storage at 60 °C for 4 weeks (an aggressive stability testing) under ambient humidity. Stability results are shown in Table 1R.

The results show stability of bromfenac for all preservatives tested, at least as good as, if not better than, the formulation with BAK. Compare with data disclosed in US Patent 8,129,431. Here, loss of bromfenac is in the range of 3-5%, with chlorhexidine showing the highest amount of loss of bromfenac (5%).

The compositions that include polyquatrenium-1 or polyquaternium-42 satisfy all three preservative efficacy criteria. The composition that includes PAPB satisfies the preservative efficacy criteria of EP-B and USP. The composition that includes sodium perborate/Dequest 2060 satisfies the preservative efficacy criteria USP.

**Table 2. Non-Limiting Preparations of the Present Invention Including Representative Surfactants**

| Ingredient | NGB-6 (%w/v) | NGB-7 (%w/v) | NGB-8 (%w/v) | NGB-9 (%w/v) |
|---|---|---|---|---|
| Bromfenac | 0.07 | 0.07 | 0.07 | 0.07 |
| Boric Acid | 1.4 | 1.4 | 1.4 | 1.4 |
| Sodium Borate | 0.74 | 0.74 | 0.74 | 0.74 |
| Sodium Sulfite | 0.2 | 0.2 | 0.2 | 0.2 |
| EDTA | 0.02 | 0.02 | 0.02 | 0.02 |
| Povidone | 1.00 | 1.00 | 1.00 | 1.00 |
| 2N NaOH | pH to 7.8 | pH to 7.8 | pH to 7.8 | pH to 7.8 |
| BAK | 0.005 | 0.005 | 0.005 | 0.005 |
| Poloxamer 407 | 0.02 | --- | --- | --- |
| Polyoxyl 40 stearate | --- | 0.15 | --- | --- |
| Octoxynol 40 | --- | --- | 0.003 | --- |
| PEG-40 hydrogenated Castor oil | --- | --- | --- | 0.02 |

**Table 2R. Results**

| Bromfenac Stability | | | | | | | | | | | | | | | | | |
|---|---|---|---|---|---|---|---|---|---|---|---|---|---|---|---|---|---|
| | | NGB-6 (BAK + Poloxamer 407) | | | | NGB-7 (BAK + Poloxyl 40 Stearate) | | | | NGB-8 (BAK + Octoxynol 40) | | | | NGB-9 (BAK + PEG-40 Castor Oil) | | | |
| | | (Initial) | | (1 Month @ 60°C) | | (Initial) | | (1 Month @ 60°C) | | (Initial) | | (1 Month @ 60°C) | | (Initial) | | (1 Month @ 60°C) | |
| Units | Component Name | S1 | S2 | S1 | S2 | S1 | S2 | S1 | S2 | S1 | S2 | S1 | S2 | S1 | S2 | S1 | S2 |
| % Label Claim | Bromfenac | 101 | 102 | 94 | 94 | 100 | 99 | 88 | 88 | 101 | 100 | 94 | 94 | 100 | 101 | 94 | 95 |
| | Total Impurities (Excluding U-II) | 0.0 | 0.0 | 0.1 | 0.1 | 0.0 | 0.0 | 0.4 | 0.4 | 0.0 | 0.0 | 0.0 | 0.1 | 0.0 | 0.0 | 0.0 | 0.0 |
| | Total Impurities | 0.0 | 0.0 | 2.2 | 2.2 | 0.0 | 0.0 | 3.7 | 4.3 | 0.0 | 0.0 | 2.4 | 2.4 | 0.0 | 0.0 | 2.3 | 2.3 |
| Dimer (Visual Inspection) | | N/A | | No Visible Red Precipitate Detected | | N/A | | No Visible Red Precipitate Detected | | N/A | | No Visible Red Precipitate Detected | | N/A | | No Visible Red Precipitate Detected | |

| | | | | | | | | | | | | | | | | | |
|---|---|---|---|---|---|---|---|---|---|---|---|---|---|---|---|---|---|
| ND=Not Detected <0.1% = Individual Impurity is less than 0.1% label claim and below reporting limit. | | | | | | | | | | | | | | | | | |

### Preservative Efficacy

**NGB-6**

| Organism | Time Intervals | | | | | | |
|---|---|---|---|---|---|---|---|
| | 0 hr | 6 hr | 24 hr | 48 hr | 7 day | 14 day | 28 day |
| *A. brasiliensis* | N/A | N/A | N/A/ | N/A | >4.10 | >4.10 | >4.10 |
| *C. Albicans* | N/A | N/A | N/A/ | N/A | >4.53 | >4.53 | >4.53 |
| *E. coli* | >4.45 | N/A | >4.45 | N/A | >4.45 | >4.45 | >4.45 |
| *S. aureus* | >4.13 | N/A | >4.13 | N/A | >4.13 | >4.13 | >4.13 |
| *P. aeruginosa* | >4.02 | N/A | >4.02 | N/A | >4.02 | >4.02 | >4.02 |

| | | | | | | | |
|---|---|---|---|---|---|---|---|
| N/A = not available because there is no requirement at this time point | | | | | | | |

**NGB-8**

| Organism | Time Intervals | | | | | | |
|---|---|---|---|---|---|---|---|
| | 0 hr | 6 hr | 24 hr | 48 hr | 7 day | 14 day | 28 day |
| *A. brasiliensis* | *N*/*A* | *N*/*A* | *N*/*A*/ | *N*/*A* | *>4.10* | *>4.10* | *>4.10* |
| *C. Albicans* | *N*/*A* | *N*/*A* | *N*/*A*/ | *N*/*A* | >*4.53* | >*4.53* | >*4.53* |
| *E. coli* | >*4.45* | *N*/*A* | >*4.45* | *N*/*A* | >*4.45* | >4.45 | >4.45 |
| *S. aureus* | >*4.13* | *N*/*A* | >*4.13* | *N*/*A* | >*4.13* | >*4.13* | >*4.13* |
| *P. aeruginosa* | *>4.02* | *N*/*A* | *>4.02* | *N*/*A* | *>4.02* | *>4.02* | *>4.02* |

**NGB-9**

| Organism | Time Intervals | | | | | | |
|---|---|---|---|---|---|---|---|
| | 0 hr | 6 hr | 24 hr | 48 hr | 7 day | 14 day | 28 day |
| *A. brasiliensis* | *N*/*A* | *N*/*A* | *N*/*A*/ | *N*/*A* | *>4.10* | *>4.10* | *>4.10* |
| *C. Albicans* | *N*/*A* | *N*/*A* | *N*/*A*/ | *N*/*A* | *>4.53* | >4.53 | >4.53 |
| *E. coli* | *>4.45* | *N*/*A* | *>4.45* | *N*/*A* | *>4.45* | >4.45 | >4.45 |
| *S. aureus* | *∼3.83* | *N*/*A* | *>4.13* | *N*/*A* | *>4.13* | *>4.13* | *>4.13* |
| *P. aeruginosa* | *>4.02* | *N*/*A* | *>4.02* | *N*/*A* | *>4.02* | *>4.02* | *>4.02* |

Bromfenac Is Chemically Stable in Compositions Comprising a Variety of Non-Ionic Surfactants.

Table 2 shows compositions comprising a variety of exemplary non-ionic surfactants other than tyloxapol: Poloxamer 407, polyoxyl 40 stearate, Octoxynol 40, and PEG-40 castor oil. Stability results are shown in Table 2R. The results show stability of bromfenac for these surfactants, comparable to tyloxapol. Compare with data on tyloxapol in US Patent 8,129,431. Here, loss of bromfenac is in the range of 5-6% with Poloxamer 407, Octoxynol 40, and PEG-40 castor oil. Loss of bromfenac of 12 % with polyoxyl 40 stearate was due to a high concentration of this surfactant, which was chosen for the test formulation. However, better bromfenac stability (comparable to that in formulation containing tyloxapol) may be achieved with lower concentrations of polyoxyl 40 stearate. Formulations NGB-6, NGB-8, and NGB-9 satisfy all three preservative efficacy criteria.

**Table 3. Non-Limiting Compositions of the Present Invention Including Representative Combinations of Surfactants and Preservatives**

| Ingredient | NGB-10 (%w/v) | NGB-1 1 (%w/v) | NGB-12 (%w/v) | NGB-13 (%w/v) | NGB-14 (%w/v) | NGB-14A (%w/v) |
|---|---|---|---|---|---|---|
| Bromfenac | 0.07 | 0.07 | 0.07 | 0.07 | 0.07 | 0.07 |
| Boric Acid | 1.4 | 1.4 | 1.4 | 1.4 | 1.4 | 1.4 |
| Sodium Borate | 0.74 | 0.74 | 0.74 | 0.74 | 0.74 | 0.74 |
| Sodium Sulfite | 0.2 | 0.2 | 0.2 | 0.2 | 0.2 | 0.2 |
| EDTA | 0.02 | 0.02 | 0.02 | 0.02 | 0.02 | 0.02 |
| Povidone | 1.00 | 1.00 | 1.00 | 1.00 | 1.00 | 1.00 |
| 2N NaOH | pH to 7.8 | pH to 7.8 | pH to 7.8 | pH to 7.8 | pH to 7.8 | pH to 8.3 |
| Polyquaternium-1 | 0.001 | 0.001 | 0.001 | 0.001 | 0.001 | 0.001 |
| Poloxamer 407 | 0.02 | --- | --- | --- | --- | --- |
| Polyoxyl 40 stearate | --- | 0.15 | --- | --- | --- | --- |
| Octoxynol 40 | --- | --- | 0.003 | --- | --- | --- |
| PEG-40 hydrogenated Castor oil | --- | --- | --- | 0.02 | --- | --- |
| Polysorbate 80 | --- | --- | --- | --- | 0.15 | 0.15 |

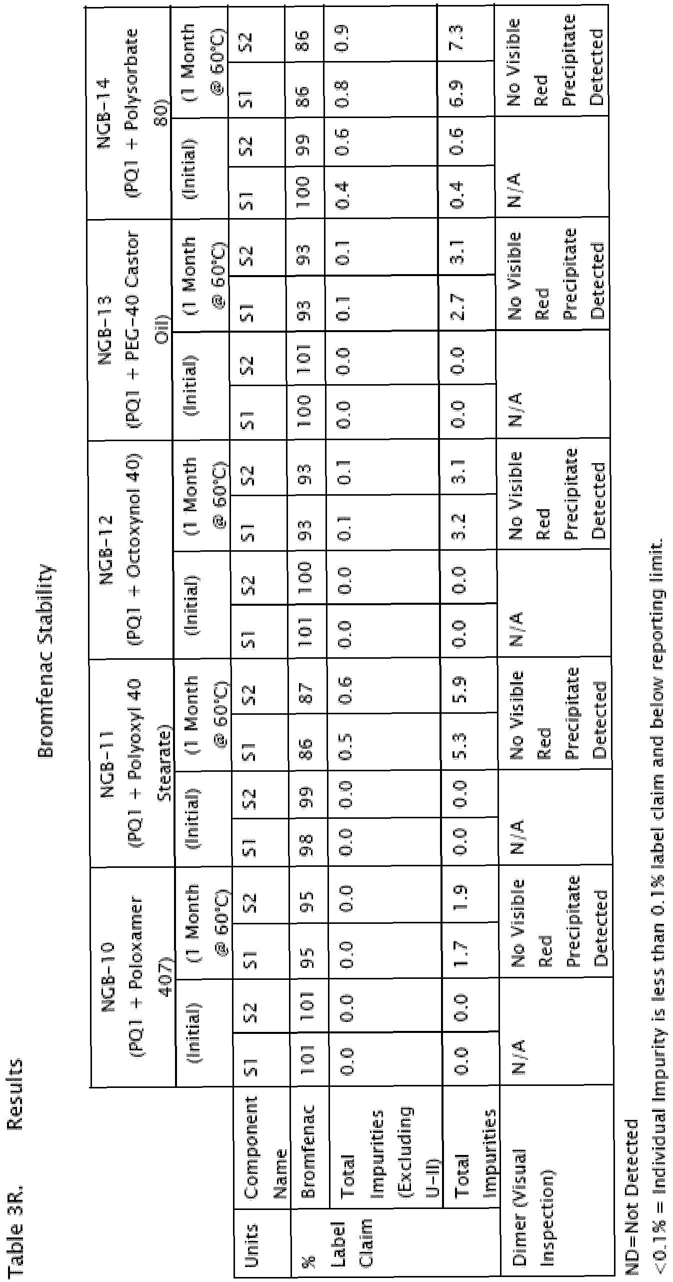

### Preservative Efficacy

**NGB-10**

| Organism | Time Intervals | | | | | | |
|---|---|---|---|---|---|---|---|
| | 0 hr | 6 hr | 24 hr | 48 hr | 7 day | 14 day | 28 day |
| *A. brasiliensis* | N/A | N/A | N/A/ | N/A | 2.02 | >4.10 | >4.10 |
| *C. Albicans* | N/A | N/A | N/A/ | N/A | >4.53 | >4.53 | >4.53 |
| *E. coli* | >4.45 | N/A | >4.45 | N/A | *>4.45* | >4.45 | >4.45 |
| *S. aureus* | >4.13 | N/A | >4.13 | N/A | >4.13 | >4.13 | >4.13 |
| *P. aeruginosa* | >4.02 | N/A | >4.02 | N/A | >4.02 | >4.02 | >4.02 |

| | | | | | | | |
|---|---|---|---|---|---|---|---|
| N/A = not available because there is no requirement at this time point | | | | | | | |

**NGB-12**

| Organism | Time Intervals | | | | | | |
|---|---|---|---|---|---|---|---|
| | 0 hr | 6 hr | 24 hr | 48 hr | 7 day | 14 day | 28 day |
| *A. brasiliensis* | N/A | N/A | N/A/ | N/A | 2.05 | >4.10 | >4.10 |
| *C. Albicans* | N/A | N/A | N/A/ | N/A | >4.53 | >4.53 | >4.53 |
| *E. coli* | >4.45 | N/A | >4.45 | N/A | >4.45 | >4.45 | >4.45 |
| *S. aureus* | >4.13 | N/A | >4.13 | N/A | >4.13 | >4.13 | >4.13 |
| *P. aeruginosa* | >4.02 | N/A | >4.02 | N/A | >4.02 | >4.02 | >4.02 |

**NGB-13**

| Organism | Time Intervals | | | | | | |
|---|---|---|---|---|---|---|---|
| | 0 hr | 6 hr | 24 hr | 48 hr | 7 day | 14 day | 28 day |
| *A. brasiliensis* | N/A | N/A | N/A/ | N/A | 3.05 | ∼3.80 | 2.92 |
| *C. Albicans* | N/A | N/A | N/A/ | N/A | >4.53 | >4.53 | >4.53 |
| *E. coli* | >4.45 | N/A | >4.45 | N/A | >4.45 | >4.45 | >4.45 |
| *S. aureus* | >4.13 | N/A | >4.13 | N/A | >4.13 | >4.13 | >4.13 |
| *P. aeruginosa* | >4.02 | N/A | >4.02 | N/A | >4.02 | >4.02 | >4.02 |

**NGB-14**

| Organism | Time Intervals | | | | | | |
|---|---|---|---|---|---|---|---|
| | 0 hr | 6 hr | 24 hr | 48 hr | 7 day | 14 day | 28 day |
| *A. brasiliensis* | N/A | N/A | N/A/ | N/A | 2.97 | 3.22 | >4.10 |
| *C. Albicans* | N/A | N/A | N/A/ | N/A | >4.53 | >4.53 | >4.53 |
| *E. coli* | >4.45 | N/A | >4.45 | N/A | >4.45 | >4.45 | >4.45 |
| *S. aureus* | >4.13 | N/A | >4.13 | N/A | >4.13 | >4.13 | >4.13 |
| *P. aeruginosa* | >4.02 | N/A | >4.02 | N/A | >4.02 | >4.02 | >4.02 |

**NGB-14A**

| Organism | Time Intervals | | | | | | |
|---|---|---|---|---|---|---|---|
| | 0 hr | 6 hr | 24 hr | 48 hr | 7 day | 14 day | 28 day |
| *A. brasiliensis* | N/A | N/A | N/A/ | N/A | 3.00 | ∼3.24 | ∼3.80 |
| *C. Albicans* | N/A | N/A | N/A/ | N/A | ∼4.11 | >4.53 | >4.53 |
| *E. coli* | ∼4.23 | N/A | >4.45 | N/A | >4.45 | >4.45 | >4.45 |
| *S. aureus* | ∼3.10 | N/A | >4.13 | N/A | >4.13 | >4.13 | >4.13 |
| *P. aeruginosa* | ∼4.02 | N/A | >4.02 | N/A | >4.02 | >4.02 | >4.02 |

Bromfenac Is Chemically Stable in Compositions Comprising a Combination of Polyquat-1 and Selected Surfactants.

Table 3 shows compositions within the scope of the present invention, comprising the preservative polyquat-1 (instead of BAK) and surfactants other than tyloxapol (Poloxamer 407, polyoxyl 40 stearate, Octoxynol 40, PEG-40 castor oil, and polysorbate 80). Stability results are shown in Table 3R. The results show stability of bromfenac in formulations containing polyquat-1 and Poloxamer 407, Octoxynol 40, or PEG-40 castor oil. The stability for these formulations is comparable to that containing BAK and tyloxapol. Compare with data on tyloxapol in US Patent 8,129,431. Here, loss of bromfenac is in the range of 5-7% with Poloxamer 407, Octoxynol 40, and PEG-40 castor oil. Loss of bromfenac of 13 % with polyoxyl 40 stearate may be due to a high concentration of this surfactant, which was chosen for the test formulation. However, better bromfenac stability (comparable to that in formulation containing tyloxapol) may be achieved with lower concentrations of polyoxyl 40 stearate. A higher bromfenac loss with polysorbate 80 is consistent with previous findings disclosed in the '431 Patent.

These results show that the combinations of polyquat-1 and other selected surfactants are novel and non-obvious alternatives to the combination of BAK and tyloxapol. It is surprising that these formulations, which do not include any polyol, satisfy all three preservative efficacy criteria with only the polyquaternium-1 preservative.

**Table 4. Additional Non-Limiting Preparations of the Present Invention Including Representative Combinations of Surfactants and Preservatives**

| Ingredient | NGB-15 (%w/v) | NGB-16 (%w/v) | NGB-1 7 (%w/v) | NGB-18 (%w/v) | NGB-19 (%w/v) |
|---|---|---|---|---|---|
| Bromfenac | 0.07 | 0.07 | 0.07 | 0.07 | 0.07 |
| Boric Acid | 1.4 | 1.4 | 1.4 | 1.4 | 1.4 |
| Sodium Borate | 0.74 | 0.74 | 0.74 | 0.74 | 0.74 |
| Sodium Sulfite | 0.2 | 0.2 | 0.2 | 0.2 | 0.2 |
| EDTA | 0.02 | 0.02 | 0.02 | 0.02 | 0.02 |
| Povidone | 1.00 | 1.00 | 1.00 | 1.00 | 1.00 |
| 2N NaOH | pH to 7.8 | pH to 7.8 | pH to 7.8 | pH to 7.8 | pH to 7.8 |
| PAPB | 0.0001 | 0.0001 | 0.0001 | 0.0001 | 0.0001 |
| Poloxamer 407 | 0.02 | --- | --- | --- | --- |
| Polyoxyl 40 stearate | --- | 0.15 | --- | --- | --- |
| Octoxynol 40 | --- | --- | 0.003 | --- | --- |
| PEG-40 hydrogenated Castor oil | --- | --- | --- | 0.02 | --- |
| Polysorbate 80 | --- | --- | --- | --- | 0.15 |

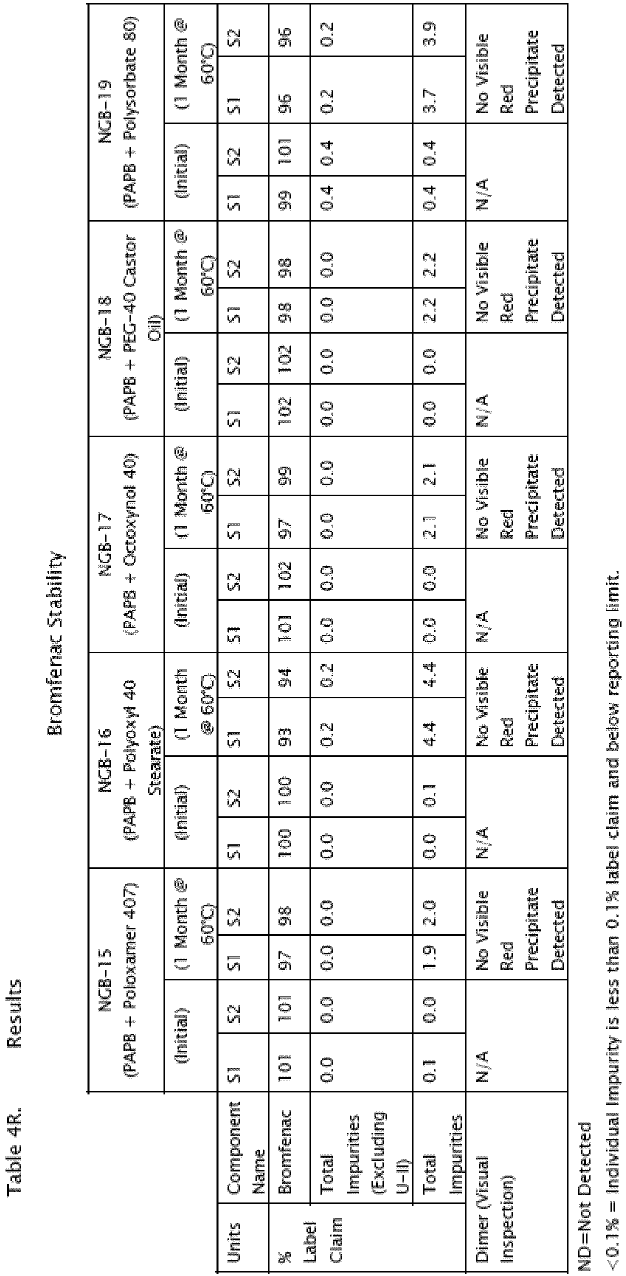

### Preservative Efficacy

**NGB-15**

| Organism | Time Intervals | | | | | | |
|---|---|---|---|---|---|---|---|
| | 0 hr | 6 hr | 24 hr | 48 hr | 7 day | 14 day | 28 day |
| *A. brasiliensis* | N/A | N/A | N/A/ | N/A | 1.88 | 3.20 | 2.94 |
| *C. Albicans* | N/A | N/A | N/A/ | N/A | >4.53 | >4.53 | >4.53 |
| *E. coli* | 0.65 | N/A | 2.52 | N/A | >4.45 | >4.45 | >4.45 |
| *S. aureus* | 0.52 | N/A | 0.60 | N/A | >4.13 | >4.13 | >4.13 |
| *P. aeruginosa* | 0.24 | N/A | 2.06 | N/A | >4.02 | >4.02 | >4.02 |

| | | | | | | | |
|---|---|---|---|---|---|---|---|
| N/A = not available because there is no requirement at this time point | | | | | | | |

**NGB-17**

| Organism | Time Intervals | | | | | | |
|---|---|---|---|---|---|---|---|
| | 0 hr | 6 hr | 24 hr | 48 hr | 7 day | 14 day | 28 day |
| *A. brasiliensis* | N/A | N/A | N/A/ | N/A | 2.16 | 2.82 | 3.10 |
| *C. Albicans* | N/A | N/A | N/A/ | N/A | >4.53 | >4.53 | >4.53 |
| *E. coli* | 0.82 | N/A | ∼4.33 | N/A | >4.45 | >4.45 | >4.45 |
| *S. aureus* | 0.06 | N/A | 0.19 | N/A | >4.13 | >4.13 | >4.13 |
| *P. aeruginosa* | 0.39 | N/A | >4.02 | N/A | >4.02 | >4.02 | >4.02 |

**NGB-18**

| Organism | Time Intervals | | | | | | |
|---|---|---|---|---|---|---|---|
| | 0 hr | 6 hr | 24 hr | 48 hr | 7 day | 14 day | 28 day |
| *A. brasiliensis* | N/A | N/A | N/A/ | N/A | 2.46 | 2.91 | 2.83 |
| *C. Albicans* | N/A | N/A | N/A/ | N/A | ∼3.93 | >4.53 | >4.53 |
| *E. coli* | 0.69 | N/A | 1.80 | N/A | >4.45 | >4.45 | >4.45 |
| *S. aureus* | 0.17 | N/A | 0.14 | N/A | >4.13 | >4.13 | >4.13 |
| *P. aeruginosa* | 0.18 | N/A | 1.37 | N/A | >4.02 | >4.02 | >4.02 |

**NGB-19**

| Organism | Time Intervals | | | | | | |
|---|---|---|---|---|---|---|---|
| | 0 hr | 6 hr | 24 hr | 48 hr | 7 day | 14 day | 28 day |
| *A. brasiliensis* | N/A | N/A | N/A/ | N/A | 2.58 | 2.65 | ∼3.30 |
| *C. Albicans* | N/A | N/A | N/A/ | N/A | ∼3.32 | >4.53 | >4.53 |
| *E. coli* | 0.83 | N/A | 2.03 | N/A | >4.45 | >4.45 | >4.45 |
| *S. aureus* | 0.19 | N/A | 0.15 | N/A | >4.13 | >4.13 | >4.13 |
| *P. aeruginosa* | 0.25 | N/A | 1.61 | N/A | >4.02 | >4.02 | >4.02 |

Bromfenac Is Chemically Stable in a Combination of PAPB (Polyaminopropyl Biguanide) and Selected Surfactants.

Table 4 shows a claimed formulation with the preservative PAPB (instead of BAK) and surfactants other than tyloxapol (Poloxamer 407, polyoxyl 40 stearate, Octoxynol 40, PEG-40 castor oil, and polysorbate 80). Stability results are shown in Table 4R.

Surprisingly, the results show good-to-excellent stability of bromfenac in formulations containing PAPB and these surfactants, even for polyoxyl 40 stearate at high concentration and polysorbate 80. These findings are non-obvious to persons skilled in the art, and patentable.

The stability for these formulations is better than for the formulation containing BAK and tyloxapol. Compare with data on tyloxapol in US Patent 8,129,431. Here, loss of bromfenac is in the range of 2-4% with Poloxamer 407, Octoxynol 40, PEG-40 castor oil, and polysorbate 80. Loss of bromfenac is higher, at 6-7% with polyoxyl 40 stearate. However, this result may be due to the high concentration of polyoxyl 40 stearate used in this formulation. Better bromfenac stability (comparable to that in formulation containing tyloxapol) may be achieved with lower concentrations of polyoxyl 40 stearate.

These results show that the combinations of the preservative PAPB and the selected nonionic surfactants are novel and non-obvious embodiments over the combination of BAK and tyloxapol. At the very low PAPB concentration of 0.0001 % w/v, these formulations can satisfy the USP preservative efficacy criteria.

**Table 5. Additional Non-Limiting Preparations of the Present Invention Including Representative Combinations of Surfactants and Preservatives**

| Ingredient | NGB-20 (%w/v) | NGB-21 (%w/v) | NGB-22 (%w/v) | NGB-23 (%w/v) | NGB-24 (%w/v) | NGB-25 (%w/v) |
|---|---|---|---|---|---|---|
| Bromfenac | 0.07 | 0.07 | 0.07 | 0.07 | 0.07 | 0.07 |
| Boric Acid | 1.4 | 1.4 | 1.4 | 1.4 | 1.4 | 1.4 |
| Sodium Borate | 0.74 | 0.74 | 0.74 | 0.74 | 0.74 | 0.74 |
| Sodium Sulfite | 0.2 | 0.2 | 0.2 | 0.2 | 0.2 | 0.2 |
| EDTA | 0.02 | 0.02 | 0.02 | 0.02 | 0.02 | 0.02 |
| Povidone | 1.00 | 1.00 | 1.00 | 1.00 | 1.00 | 1.00 |
| 2N NaOH | pH to 7.8 | pH to 7.8 | pH to 7.8 | pH to 7.8 | pH to 7.8 | pH to 7.8 |
| Sodium | 0.02 | 0.02 | 0.02 | 0.02 | 0.02 | 0.02 |
| Perborate Dequest 2060^{a} | 0.005 | 0.005 | 0.005 | 0.005 | 0.005 | 0.005 |
| Poloxamer 407 | 0.02 | --- | --- | --- | --- | --- |
| Polyoxyl 40 stearate | --- | 0.15 | --- | --- | --- | --- |
| Octoxynol 40 | --- | --- | 0.003 | --- | --- | --- |
| PEG-40 hydrogenated Castor oil | --- | --- | --- | 0.02 | --- | --- |
| Polysorbate 80 | --- | --- | --- | --- | 0.15 | --- |

| | | | | | | |
|---|---|---|---|---|---|---|
| ^{a}Perborate generates a low concentration of hydrogen peroxide that requires phosphonates for stability | | | | | | |

### Preservative Efficacy

**NGB-20**

| Organism | Time Intervals | | | | | | |
|---|---|---|---|---|---|---|---|
| | 0 hr | 6 hr | 24 hr | 48 hr | 7 day | 14 day | 28 day |
| *A. brasiliensis* | N/A | N/A | N/A/ | N/A | 2.04 | 2.13 | 2.80 |
| *C. Albicans* | N/A | N/A | N/A/ | N/A | ∼3.61 | >4.53 | >4.53 |
| *E. coli* | 0.74 | N/A | 2.56 | N/A | ∼4.45 | >4.45 | >4.45 |
| *S. aureus* | 0.11 | N/A | 0.20 | N/A | ∼4.13 | >4.13 | >4.13 |
| *P. aeruginosa* | 0.47 | N/A | 2.10 | N/A | ∼4.02 | >4.02 | >4.02 |

| | | | | | | | |
|---|---|---|---|---|---|---|---|
| N/A = not available because there is no requirement at this time point | | | | | | | |

**NGB-22**

| Organism | Time Intervals | | | | | | |
|---|---|---|---|---|---|---|---|
| | 0 hr | 6 hr | 24 hr | 48 hr | 7 day | 14 day | 28 day |
| *A. brasiliensis* | N/A | N/A | N/A/ | N/A | 2.02 | 2.16 | 2.77 |
| *C. Albicans* | N/A | N/A | N/A/ | N/A | ∼3.61 | >4.53 | >4.53 |
| *E. coli* | 0.71 | N/A | 2.62 | N/A | ∼4.45 | >4.45 | >4.45 |
| *S. aureus* | 0.13 | N/A | >4.130.16 | N/A | ∼4.13 | >4.13 | >4.13 |
| *P. aeruginosa* | 0.30 | N/A | 2.22 | N/A | >4.02 | >4.02 | >4.02 |

**NGB-23**

| Organism | Time Intervals | | | | | | |
|---|---|---|---|---|---|---|---|
| | 0 hr | 6 hr | 24 hr | 48 hr | 7 day | 14 day | 28 day |
| *A. brasiliensis* | N/A | N/A | N/A/ | N/A | 2.04 | 2.16 | 2.66 |
| *C. Albicans* | N/A | N/A | N/A/ | N/A | ∼3.50 | >4.53 | >4.53 |
| *E. coli* | 0.79 | N/A | 2.56 | N/A | >4.45 | >4.45 | >4.45 |
| *S. aureus* | 0.16 | N/A | 0.17 | N/A | ∼4.13 | >4.13 | >4.13 |
| *P. aeruginosa* | 0.36 | N/A | 2.10 | N/A | >4.02 | >4.02 | >4.02 |

**NGB-25**

| Organism | Time Intervals | | | | | | |
|---|---|---|---|---|---|---|---|
| | 0 hr | 6 hr | 24 hr | 48 hr | 7 day | 14 day | 28 day |
| *A. brasiliensis* | N/A | N/A | N/A/ | N/A | 2.10 | 2.63 | 2.89 |
| *C. Albicans* | N/A | N/A | N/A/ | N/A | ∼3.58 | >4.53 | >4.53 |
| *E. coli* | 0.78 | N/A | 2.56 | N/A | ∼4.33 | >4.45 | >4.45 |
| *S. aureus* | 0.12 | N/A | 0.23 | N/A | ∼3.70 | >4.1 3 | >4.13 |
| *P. aeruginosa* | 0.44 | N/A | 2.14 | N/A | >4.02 | >4.02 | >4.02 |

Bromfenac Is Chemically Stable in a Combination of Perborate, a Chelating Agent, and Selected Surfactants.

Table 5 shows a claimed formulation with the preservative combination of perborate (instead of BAK) and the chelating agent Dequest® 2060, and surfactants other than tyloxapol (Poloxamer 407, polyoxyl 40 stearate, Octoxynol 40, PEG-40 castor oil, and polysorbate 80).

Stability results are shown in Table 5R.

Stability of bromfenac in formulations containing perborate/Dequest® 2060 and Poloxamer 407, Octoxynol 40, or PEG-40 castor oil is even somewhat better than for the formulation containing BAK and tyloxapol. Compare with data on tyloxapol in US Patent 8,129,431. Here, loss of bromfenac is in the range of 4-8% with Poloxamer 407, Octoxynol 40, and PEG-40 castor oil. Loss of bromfenac is higher, at 14-15% with polyoxyl 40 stearate. However, this result may be due to the high concentration of polyoxyl 40 stearate used in this formulation. Better bromfenac stability (comparable to that in formulation containing tyloxapol) may be achieved with lower concentrations of polyoxyl 40 stearate.

Loss of bromfenac is 13-14% with polysorbate 80. This is consistent with the results of benzalkonium chloride ("BAK")/polysorbate 80 disclosed in the '431 Patent. In addition, it is suspected that polysorbate 80 (having a saccharide moiety) may be readily degraded by an oxidizing agent like perborate.

Table 5R (experiment NGB-25) shows that without polysorbate 80, the stability of bromfenac in the formulation containing perborate (bromfenac loss of 5-6%) is surprisingly even somewhat better than that in the formulation containing BAK/tyloxapol.

These results show that the combinations of the preservative perborate/chelating agent (such as Dequest® 2060) and Poloxamer 407, Octoxynol 40, or PEG-40 castor oil are novel and non-obvious over the combination of BAK and tyloxapol. At a low concentration of 0.02 %w/v of sodium perborate, these formulations can satisfy the USP preservative efficacy criteria.

**Table 6. Study of Effect of Mannitol (a Polyol) or Tyloxapol (a Polyether) on the Efficacy of Polyquaternium-1 (a Preservative) in a Composition Comprising Diclofenac and Boric Acid**

| Ingredient | DBP-1 (%w/v) | DBP-2 (%w/v) | DBP-3 (%w/v) | DBP-4 (%w/v) | DBP-5 (%w/v) | DBP-6 (%w/v) |
|---|---|---|---|---|---|---|
| Sodium Diclofenac | 0.1 | 0.1 | 0.1 | 0.1 | 0.1 | 0.1 |
| HPMC (E4M) | 0.1 | 0.1 | 0.1 | 0.1 | 0.1 | 0.1 |
| Tromethamine | 2.0 | 2.0 | 2.0 | 2.0 | 2.0 | 2.0 |
| Boric Acid | 1.2 | 1.2 | 1.2 | 1.2 | 1.2 | 1.2 |
| Vitamin E TPGS | 3.0 | 3.0 | 3.0 | 3.0 | 3.0 | 3.0 |
| Mannitol | 3.5 | 3.5 | --- | --- | --- | --- |
| Polyquaternium-1 | 0.005 | 0.005 | 0.005 | 0.005 | 0.005 | 0.005 |
| Tyloxapol | --- | --- | --- | --- | 0.02 | 0.02 |
| HCl/NaOH | pH to 7.4 | pH to 7.8 | pH to 7.4 | pH to 7.8 | pH to 7.4 | pH to 7.8 |
| Purified Water | qs to 100% | qs to 100% | qs to 100% | qs to 100% | qs to 100% | qs to 100% |

| | | | | | | |
|---|---|---|---|---|---|---|
| *Test for USP & EP antimicrobial effectiveness only | | | | | | |

**Table 6R. Preservative Efficacy**

| DBP-1: Diclofenac + Mannitol + PQ-1 pH 7.4 | | | | | | | |
|---|---|---|---|---|---|---|---|
| Organism | Time Intervals | | | | | | |
| | 0 hr | 6 hr | 24 hr | 48 hr | 7 day | 14 day | 28 day |
| *A. brasiliensis* | 0.02 | 0.06 | 2.12 | 2.99 | 3.10 | ∼3.79 | ∼3.42 |
| *C. Albicans* | 1.01 | 2.99 | >4.51 | >4.51 | >4.51 | >4.51 | >4.51 |
| *E. coli* | 2.65 | >4.24 | >4.24 | >4.24 | >4.24 | >4.24 | >4.24 |
| *S. aureus* | ∼3.43 | >4.49 | >4.49 | >4.49 | >4.49 | >4.49 | >4.49 |
| *P. aeruginosa* | >4.6 4 | >4.64 | >4.64 | >4.64 | >4.64 | >4.64 | >4.64 |
| | | | | | | | |

| DBP-2: Diclofenac + Mannitol + PQ-1 pH 7.8 | | | | | | | |
|---|---|---|---|---|---|---|---|
| Organism | Time Intervals | | | | | | |
| | 0 hr | 6 hr | 24 hr | 48 hr | 7 day | 14 day | 28 day |
| *A. brasiliensis* | 0.05 | 0.09 | 1.35 | 2.82 | 2.28 | 2.39 | 2.59 |
| *C*. *Albicans* | 0.83 | 3.06 | >4.51 | >4.51 | >4.51 | >4.51 | >4.51 |
| *E. coli* | 3.06 | >4.24 | >4.24 | >4.24 | >4.24 | >4.24 | >4.24 |
| *S. aureus* | ∼3.52 | >4.49 | >4.49 | >4.49 | >4.49 | >4.49 | >4.49 |
| *P. aeruginosa* | >4.64 | >4.64 | >4.64 | >4.64 | >4.64 | >4.64 | >4.64 |
| | | | | | | | |

| DBP-3: Diclofenac + PQ-1 pH 7.4 (No Mannitol) | | | | | | | |
|---|---|---|---|---|---|---|---|
| Organism | Time Int er vals | | | | | | |
| | 0 hr | 6 hr | 24 hr | 48 hr | 7 day | 14 day | 28 day |
| *A. brasiliensis* | 0.03 | 0.34 | 2.01 | ∼4.01 | 3.05 | 2.95 | 2.61 |
| *C. Albicans* | ∼3.48 | >4.51 | >4.51 | >4.51 | >4.51 | >4.51 | >4.51 |
| *E. coli* | ∼3.11 | >4.24 | >4.24 | >4.24 | >4.24 | >4.24 | >4.24 |
| *S. aureus* | ∼3.37 | >4.49 | >4.49 | >4.49 | >4.49 | >4.49 | >4.49 |
| *P. aeruginosa* | >4.64 | >4.64 | >4.64 | >4.64 | >4.64 | >4.64 | >4.64 |

| DBP-4: Diclofenac + PQ-1 pH 7.8 (No Mannitol) | | | | | | | |
|---|---|---|---|---|---|---|---|
| Organism | Time Intervals | | | | | | |
| | 0 hr | 6 hr | 24 hr | 48 hr | 7 day | 14 day | 28 day |
| *A. brasiliensis* | 0.01 | 0.93 | 2.04 | 3.04 | 2.12 | 1.90 | 0.97 |
| *C*. *Albicans* | >4.51 | >4.51 | >4.51 | >4.51 | >4.51 | >4.51 | >4.51 |
| *E. coli* | ∼3.31 | >4.24 | >4.24 | >4.24 | >4.24 | >4.24 | >4.24 |
| *S. aureus* | ∼3.79 | >4.49 | >4.49 | >4.49 | >4.49 | >4.49 | >4.49 |
| *P. aeruginosa* | >4.64 | >4.64 | >4.64 | >4.64 | >4.64 | >4.64 | >4.64 |
| | | | | | | | |

| DBP-5: Diclofenac + Tyloxapol + PQ-1 pH 7.4 | | | | | | | |
|---|---|---|---|---|---|---|---|
| Organism | Time Intervals | | | | | | |
| | 0 hr | 6 hr | 24 hr | 48 hr | 7 day | 14 day | 28 day |
| *A. brasiliensis* | 0.06 | 1.19 | 2.21 | 2.96 | 3.06 | 2.93 | 1.08 |
| *C. Albicans* | ∼3.32 | >4.51 | >4.51 | >4.51 | >4.51 | >4.51 | >4.51 |
| *E. coli* | 2.73 | >4.24 | >4.24 | >4.24 | >4.24 | >4.24 | >4.24 |
| *S. aureus* | 3.40 | >4.49 | >4.49 | >4.49 | >4.49 | >4.49 | >4.49 |
| *P. aeruginosa* | ∼4.16 | >4.64 | >4.64 | >4.64 | >4.64 | >4.64 | >4.64 |
| | | | | | | | |

| DBP-6: Diclofenac + Tyloxapol + PQ-1 pH 7.8 | | | | | | | |
|---|---|---|---|---|---|---|---|
| Organism | Time Intervals | | | | | | |
| | 0 hr | 6 hr | 24 hr | 48 hr | 7 day | 14 day | 28 day |
| *A. brasiliensis* | 0.01 | 1.03 | 2.70 | 2.98 | 2.05 | 1.95 | 1.34 |
| *C*. *Albicans* | >4.51 | >4.51 | >4.51 | >4.51 | >4.51 | >4.51 | >4.51 |
| *E. coli* | ∼3.43 | >4.24 | >4.24 | >4.24 | >4.24 | >4.24 | >4.24 |
| *S. aureus* | ∼3.69 | >4.49 | >4.49 | >4.49 | >4.49 | >4.49 | >4.49 |
| *P. aeruginosa* | >4.64 | >4.64 | >4.64 | >4.64 | >4.64 | >4.64 | >4.64 |

Table 6 shows some formulations comprising diclofenac (an non-steroidal anti-inflammatory agent) and polyquaternium-1 within the scope of the present invention and compared these formulations to those shown in US Patent 5,603,929 (issued to Desai). Table 6R shows the preservative efficacy results of the formulations shown in Table 6. Table 6R shows the log reduction in the counts of the microorganisms compared to the initial counts.

The following table (from Desai, US Pat. 5,603,929) shows the criteria to pass the preservative efficacy testing under US Pharmacopeia XXII ("USP XXII"), European Pharmacopeia A ("EP-A"), and European Pharmacopeia B ("EP-B") as existing at the time of Desai's disclosure. EP-A has the most stringent criteria.

| Log Reduction of Organism Population | | | |
|---|---|---|---|
| Time Pull | USP | *Ph. Eur.* A (Target) | *Ph. Eur.* B (Min) |
| For Bacteria: | | | |
| 6 hours | - | 2 | - |
| 24 hours | - | 3 | 1 |
| 7 days | - | - | 3 |
| 14 days | 3 | - | - |
| 28 days | NI | NR | NI |

| For Fungi: | | | |
|---|---|---|---|
| 7 days | - | 2 | - |
| 14 days | NI | - | 1 |
| 28 days | NI | NI | NI |

| | | | |
|---|---|---|---|
| NR = no organisms recovered NI = no increase compared to the previous count - = not required at this designated time | | | |

DBP-1 is Desai's formulation B or C with diclofenac as the active ingredient. The concentration of polyquat-1 of 0.005% is what typically is used with this preservative. Note that Formulation A has the much higher concentration of 4% polyquat-1, a level of greater than 1000 times that typically used in commercial ophthalmic products. The experiments at 0.005% polyquat-1 show the importance of mannitol in achieving Desai's purpose.

DBP-2 is the same as DBP-1 , except pH of 7.8, to discern any effect of pH.

DBP-3 and DBP-4 correspond to DBP-1 and DBP-2, respectively, without mannitol. The results for these formulations are to show the requirement of mannitol in Desai's formulation.

DBP-5 and DBP-6 correspond to DBP-1 and DBP-2, respectively, without mannitol, but with tyloxapol. Tyloxapol is not a polyol (instead, it is a polyether having Formula II shown above and is within the scope of the present invention).

### Conclusion:

Generally, everything else being kept constant, a lower pH of 7.4 is more effective than a pH of 7.8. However, whether a formulation meets the stated preservative efficacy criteria does not depend on pH in the range of 7.4-7.8.

Only formulations containing polyquat-1, boric acid, and mannitol (DBP-1 and DBP-2) meet all three preservative efficacy criteria, as they existed at the time of Desai's disclosure.

None of the formulations without mannitol (DBP-3 through DBP-6) meets any preservative efficacy criteria, as they existed at the time of Desai's disclosure because the population of the fungus *A*. *brasiliensis* shows an increase from the previous time point. Specifically, these formulations do not meet the USP XXII and EP-B criteria because the population of *A*. *brasiliensis* at 28 days is higher than that at 14 days. They do not meet the EP-A criteria because the population of *A*. *brasiliensis* at 28 days is higher than that at 7 days. In other words, excluding mannitol from Desai's formulations renders the latter unsuitable for their purpose; i.e., meeting both USP XXII and EP preservative efficacy criteria.

Nothing in Desai's disclosure suggests that a composition with polyquat-1 and without a polyol such as mannitol that is within the scope of the present invention would meet at least the USP preservative efficacy criteria at the time of the present invention.

Therefore, compositions within the scope of the present invention are novel and inventive over Desai's composition.

### Industrial Applicability

The aqueous liquid preparations of the present invention in the form of eye drops are useful for the treatment of ocular inflammation having diverse etiology, including blepharitis, conjunctivitis, scleritis, and postoperative inflammation. Such preparations are also useful as nasal drop for the treatment of, for example, allergic rhinitis and inflammatory rhinitis (e.g., chronic rhinitis, hypertrophic rhinitis, nasal polyp, etc.)

## Claims

1. An aqueous liquid composition comprising: (a) 2-amino-3-(4-bromobenzoyl)phenylacetic acid or a pharmacologically acceptable salt thereof or a hydrate thereof; (b) a non-ionic surfactant; (c) water; and (d) an ophthalmically acceptable preservative selected from the group consisting of polyaminopropyl biguanide ("PAPB"), polyquatemium-1, perborate, and mixtures thereof; wherein the non-ionic surfactant is selected from the group consisting of Poloxamer 407, Octoxynol 40, PEG40 hydrogenated castor oil, and mixtures thereof; wherein at least 90 percent of the original amount of said 2-amino-3-(4-bromobenzoyl)phenylacetic acid or pharmacologically acceptable salt thereof or hydrate thereof remains in the composition after storage at 60 °C, for 4 weeks; and wherein the composition satisfies preservative efficacy acceptance criteria of US Pharmacopeia 35 (2012).

2. The composition according to claim 1, wherein the concentration of the non-ionic surfactant is in the range from 0.005 w/v % to 0.12 w/v %.

3. The composition according to claim 1, wherein the pH of the aqueous liquid preparation is within a range of 7.3 to 8.5.

4. The composition according to any one of claims 1-3, wherein the composition does not include any polyol.

5. The composition of claim 4, wherein the polyol is mannitol.

6. A method for inhibiting decrease in preservative efficacy of an ophthalmically acceptable preservative in an aqueous composition that comprises: (a) 2-amino-3-(4-bromobenzoyl)phenylacetic acid or a pharmacologically acceptable salt thereof or a hydrate thereof; and (b) an ophthalmically acceptable preservative selected from the group consisting of polyaminopropyl biguanide ("PAPB"), polyquatemium-1, perborate, and mixtures thereof; which method comprises incorporating a non-ionic surfactant into the aqueous liquid composition; wherein the non-ionic surfactant is selected from the group consisting of Poloxamer 407, Octoxynol 40, PEG40 hydrogenated castor oil, and mixtures thereof, and wherein the preservative efficacy satisfies the preservative efficacy acceptance criteria of US Pharmacopeia 35 (2012).

7. The method of claim 6, wherein the non-ionic surfactant is present in the preparation at a concentration in the range from 0.005 w/v % to 0.12 w/v %.

## Patentansprüche

1. Eine wässrige, flüssige Zusammensetzung umfassend: (a) 2-Amino-3-(4-brombenzoyl)phenylessigsäure oder ein pharmakologisch annehmbares Salz davon oder ein Hydrat davon; (b) ein nichtionisches Tensid; (c) Wasser; and (d) ein ophthalmisch verträgliches Konservierungsmittel ausgewählt aus der Gruppe bestehend aus Polyaminopropylbiguanid ("PAPB"), Polyquatemium-1, Perborat, und Mischungen davon; wobei das nichtionische Tensid ausgewählt ist aus der Gruppe bestehend aus Poloxamer 407, Octoxynol 40, PEG40 hydriertes Rizinusöl, und Mischungen davon; wobei mindestens 90 Prozent der ursprünglichen Menge der besagten 2-Amino-3-(4-brombenzoyl)phenylessigsäure oder eines pharmakologisch annehmbaren Salzes davon oder eines Hydrats davon nach einer Lagerung bei 60 °C für 4 Wochen in der Zusammensetzung verbleibt; und wobei die Zusammensetzung die Zulassungskriterien hinsichtlich der Konservierungsmittelwirksamkeit gemäß US Pharmacopeia 35 (2012) erfüllt.

2. Die Zusammensetzung gemäß Anspruch 1, wobei die Konzentration des nichtionischen Tensids im Bereich von 0.005 w/v % bis 0.12 w/v % ist.

3. Die Zusammensetzung gemäß Anspruch 1, wobei der pH-Wert der wässrigen, flüssigen Zubereitung im Bereich von 7,3 bis 8,5 ist.

4. Die Zusammensetzung gemäß einem der Ansprüche 1-3, wobei die Zusammensetzung kein Polyol enthält.

5. Die Zusammensetzung gemäß Anspruch 4, wobei das Polyol Mannitol ist.

6. Ein Verfahren zur Hemmung des Abfalls der Konservierungswirkung eines ophthalmisch verträglichen Konservierungsmittels in einer wässrigen Zusammensetzung umfassend: (a) 2-Amino-3-(4-brombenzoyl)phenylessigsäure oder ein pharmakologisch annehmbares Salz davon oder ein Hydrat davon; und (b) ein ophthalmisch verträgliches Konservierungsmittel ausgewählt aus der Gruppe bestehend aus Polyaminopropylbiguanid ("PAPB"), Polyquatemium-1, Perborat, und Mischungen davon; wobei das Verfahren das Zusetzen eines nichtionischen Tensids in die wässrige, flüssige Zusammensetzung umfasst; wobei das nichtionische Tensid ausgewählt ist aus der Gruppe bestehend aus Poloxamer 407, Octoxynol 40, PEG40 hydriertes Rizinusöl, und Mischungen davon, und wobei das Konservierungsmittel die Zulassungskriterien hinsichtlich der Konservierungsmittelwirksamkeit gemäß US Pharmacopeia 35 (2012) erfüllt.

7. Das Verfahren gemäß Anspruch 6, wobei das nichtionische Tensid in einer Konzentration im Bereich von 0.005 w/v % bis 0.12 w/v % vorhanden ist.

## Revendications

1. Composition liquide aqueuse comprenant: (a) de l'acide 2-amino-3-(4-bromobenzoyl)phénylacétique ou un sel pharmacologiquement acceptable de celui-ci ou un hydrate de celui-ci; (b) un surfactant non ionique; (c) de l'eau; et (d) un conservateur acceptable sur le plan ophtalmique sélectionné dans le groupe constitué du polyaminopropyl biguanide (« PAPB »), du polyquatemium-1, du perborate, et de mélanges de ceux-ci; dans laquelle le surfactant non ionique est sélectionné dans le groupe constitué du Poloxamer 407; de l'Octoxynol 40, de l'huile de ricin hydrogénée PEG40, et de mélanges de ceux-ci; dans laquelle au moins 90 % de la quantité initiale dudit acide 2-amino-3-(4-bromobenzoyl)phénylacétique ou sel pharmacologiquement acceptable de celui-ci ou hydrate de celui-ci restent dans la composition après un stockage à 60 °C pendant 4 semaines ; et dans laquelle la composition satisfait au critère d'acceptation de l'efficacité de conservation de la Pharmacopée US 35 (2012).

2. Composition selon la revendication 1, dans laquelle la concentration en surfactant non ionique se trouve dans la plage de 0,005 % p/v à 0,12 % p/v.

3. Composition selon la revendication 1, dans laquelle le pH de la préparation liquide aqueuse se trouve dans la plage de 7,3 à 8,5.

4. Composition selon l'une quelconque des revendications 1 - 3, dans laquelle la composition ne comprend pas de polyol.

5. Composition selon la revendication 4, dans laquelle le polyol est le mannitol.

6. Procédé d'inhibition d'une réduction de l'efficacité de conservation d'un conservateur acceptable sur le plan ophtalmique dans une composition aqueuse qui comprend : (a) de l'acide 2-amino-3-(4-bromobenzoyl)phénylacétique ou un sel pharmacologiquement acceptable de celui-ci ou un hydrate de celui-ci; et (b) un conservateur acceptable sur le plan ophtalmique sélectionné dans le groupe constitué du polyaminopropyl biguanide (« PAPB »), du polyquatemium-1, du perborate, et de mélanges de ceux-ci; ledit procédé comprenant l'incorporation d'un surfactant non ionique dans la composition liquide aqueuse ; dans lequel le surfactant non ionique est sélectionné dans le groupe constitué du Poloxamer 407; de l'Octoxynol 40, de l'huile de ricin hydrogénée PEG40, et de mélanges de ceux-ci, et dans lequel l'efficacité de conservation satisfait au critère d'acceptation de l'efficacité de conservation de la Pharmacopée US 35 (2012).

7. Procédé selon la revendication 6, dans lequel le surfactant non ionique est présent dans la préparation en une concentration dans la plage de 0,005 % p/v à 0,12 % p/v.
